# EUROPEAN PATENT SPECIFICATION

(11) **EP 3 345 580 B1**
(45) Date of publication and mention of the grant of the patent: **31.12.2025**
(21) Application number: 16841961.2
(22) Date of filing: 01.09.2016
(51) Int. Cl.: A61F 9/007

(54) **VITREOUS BODY SURGICAL PROBE**
GLASKÖRPERCHIRURGIESONDE
SONDE CHIRURGICALE DE CORPS VITRÉ

(30) Priority: 01.09.2015 JP 2015171769
(43) Date of publication of application: 11.07.2018
(73) Proprietor: Mani, Inc., Tochigi 321-3231 (JP)
(72) Inventor: MURAKAMI, Etsuo, Utsunomiya-shi Tochigi 321-3231 (JP)
(74) Representative: Isarpatent
(86) International application number: PCT/JP2016/075638
(87) International publication number: WO 2017/038932

(56) References cited:
- EP-A1- 2 754 427
- JP-A- 2009 525 782
- JP-A- 2013 052 168
- JP-A- H11 137 594
- US-A- 5 047 008
- US-A1- 2004 204 732
- US-A1- 2013 053 759
- US-A1- 2013 053 759

## Description

### Technical Field

The present invention relates to a vitreous body surgical probe used in ophthalmic surgery.

### Background Art

A vitreous body surgical probe used in ophthalmic surgery is used for cutting and removing from an eyeball a jelly-like vitreous body and/or a proliferative membrane (hereafter referred to as 'vitreous body etc.') on the retina generated through denaturation of the vitreous body (see Patent Documents 1 and 2, for example).

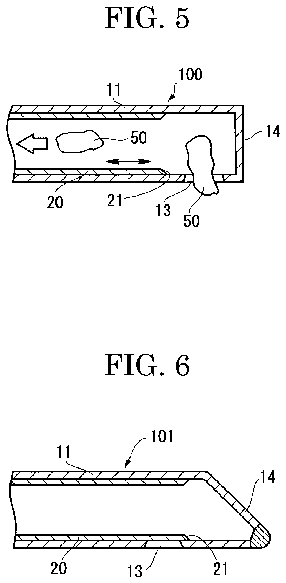

FIG. 5 shows a cross-section of a conventional vitreous body surgical probe. Such a conventional vitreous body surgical probe 100 includes a probe main body 11 having a form with a sealed, pipe tip surface 14, and a cutter 20, which has a blade part 21 at a tip part; wherein the blade part 21 is slidable in the axial direction on the inner surface of the probe main body 11 with continuous contact on that surface.

An opening 13 is provided in a side near the tip of the probe main body 11, and vitreous bodies etc. 50 are sucked in through the opening. At this time, the vitreous bodies 50 are cut when the cutter 20 slides and the blade part 21 passes by the opening 21, and the vitreous bodies etc. 50 that are cut into small pieces are sucked in at the back side of the probe (left side of FIG. 5) and collected.

It is preferable that the vitreous body surgical probe 100 has a short distance between the tip surface 14 and the opening 13. This is because the vitreous bodies etc. 50 are either near the retina or are floating in the vicinity of the retina, and provision of the opening 13 as close to the retina as possible allows reduction of residual vitreous bodies etc. 50. Moreover, the tip surface 14 is preferably a flat surface without any protrusions so that the probe main body 11 does not touch and damage the retina.

Furthermore, a vitreous body surgical probe 101 having a slanted tip surface 14 is used for bringing the probe main body 11 closer to the retina (see Patent Document 2, for example). FIG. 6 shows a cross-section of the conventional vitreous body surgical probe with a slanted tip surface.

Slanting the tip surface 14 as such may bring the probe main body 11 closer to the retina, thereby making it easier to suck in the vitreous bodies etc. 50 in the vicinity of the retina. However, since this cutter 20 may rotate when sliding along the inner side of the probe main body 11, provision of the opening 13 on the very end part is impossible. Therefore, it does not reach the tip side of the slanted surface 14. That is, the opening 13 should be provided to the position having a certain distance to the slanted surface 14 to which the blade part 21 of the cutter 20 reaches.

### Prior Art Documents

### Patent Documents

[Patent Document 1] JP 2009-511169
[Patent Document 2] JP 2014-42703A
US 2013/0053759 A1 describes an enhanced flow vitrectomy probe with a body, a needle, a cutter and a stiffening sleeve.
EP 2 754 427 A1 describes a surgical instrument with a rigid probe. An inner tube portion has a cutter and a trabecular meshwork can be sucked in from a hole portion and cut by the cutter.

### DISCLOSURE OF INVENTION

### Problem to be Solved by the Invention

In light of the problem, the present invention aims to provide a vitreous body surgical probe that includes a probe main body having sufficient bending rigidity obtained by an end part having an approximately tube shape and a smaller diameter than the probe main body, and that secures sufficient suction force, and that can make it easy to collect vitreous bodies etc. in the vicinity of the retina, which are difficult to reach using the conventional vitreous body surgical probe.

### Solution to the Problem

The present application provides a vitreous body surgical probe, as defined in the appended claim set.

### Advantageous Effect of the Invention

Use of the vitreous body surgical probe according to the present invention, which secures sufficient bending rigidity and suction force and has an end part small in diameter, brings about beneficial effects that allow easy collection of vitreous bodies etc. in the vicinity of the retina.

### BRIEF DESCRIPTION OF DRAWINGS

FIG. 1 shows a vitreous body surgical probe, wherein FIG. 1(a) is a side view and FIG. 1(b) is a front view;
FIG. 2 is a drawing explaining a usage of the vitreous body surgical probe;
FIG. 3 shows cross-sections illustrating an embodiment of the vitreous body surgical probe, wherein FIG. 3(a) illustrates a state where a cutter is pulled back, and FIG. 3(b) illustrates a state where a vitreous body or the like is cut;
FIG. 4 shows cross-sections illustrating an embodiment of another vitreous body surgical probe, wherein FIG. 4(a) illustrates a state where a cutter is pulled back, and FIG. 4(b) illustrates a state where a vitreous body or the like is cut;
FIG. 5 shows a cross-section of a conventional vitreous body surgical probe; and
FIG. 6 shows a cross-section of the conventional vitreous body surgical probe with a slanted tip surface.

### DESCRIPTION OF EMBODIMENTS

An embodiment according to the present invention is described below with reference to accompanying drawings.

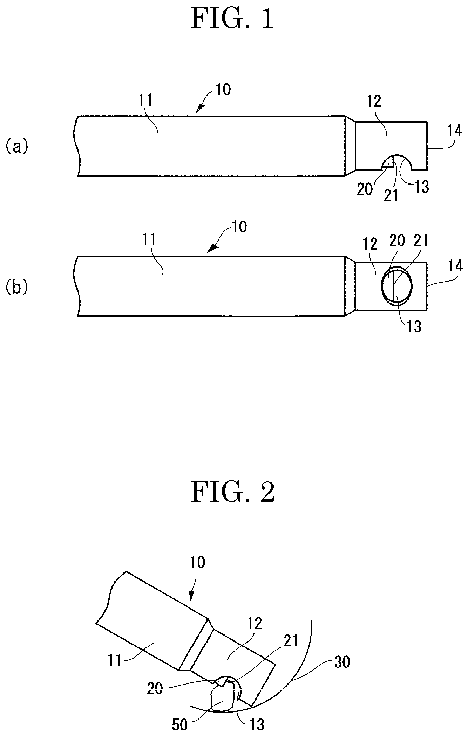

FIG. 1 shows a vitreous body surgical probe of the present invention, wherein FIG. 1(a) is a side view and FIG. 1(b) is a front view. A vitreous body surgical probe 10 has an appearance of having a pipe-shaped probe main body 11 and an end part 12 connected to each other where the end part includes an opening 13.

The end part 12 has an approximately tube shape with a sealed, flat tip surface 14, and the diameter of the end part 12 is smaller than that of the probe main body 11. As for materials for forming such a vitreous body surgical probe 10, a conventionally-used 27 gauge pipe may be used for the probe main body 11, and a 29 gauge pipe may be used for the end part 12, although not limited to this combination of materials.

Vitreous bodies etc. in the vicinity of the opening are cut when a cutter 20 slides along the inner surface of the probe main body 11 and the end part 12, and a blade part 21 provided on the tip of the cutter 20 passes by an opening 13, making it easy to collect the vitreous bodies etc.

FIG. 2 is a drawing explaining a usage of the vitreous body surgical probe. Since the end part 12 of the vitreous body surgical probe 10 according to the present invention has a smaller diameter than the probe main body 11, the opening 13 provided in the end part 12 can be provided near the retina 30. This results in a structure allowing easy suction of a vitreous body near the retina 30 or a proliferative membrane (the vitreous body etc. 50) on the retina generated through denaturation of the vitreous body. Note that the opening 13 can be provided near the retina if the probe main body 11 is made of the same thin material as the end part 12. In that case, the probe main body 11 becomes too thin, thereby making the bending rigidity weaker and the suction force weaker than in the past. Moreover, when collecting the vitreous bodies etc. 50, the probe may become clogged, thereby reducing the suction force. As a result, using a thinner material for the probe main body 11 than that used in the past is impossible in terms of performance.

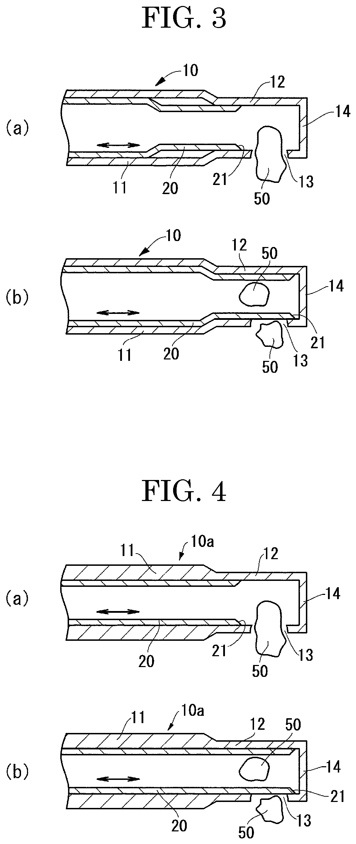

FIG. 3 shows cross-sections illustrating an embodiment of the vitreous body surgical probe, wherein FIG. 3(a) illustrates a state where a cutter is pulled back, and FIG. 3(b) illustrates a state where a vitreous body or the like is cut. The vitreous body surgical probe 10 is used when penetrating an eyeball. The diameter of the probe main body 11 is thus quite small, such as a 27 gauge. Therefore, as mentioned before, when suctioning in the vitreous bodies etc. 50 through the opening 13, cutting the vitreous bodies etc. 50 into small pieces using the cutter 20 that slides along the inner surface is required.

On the other hand, the opening 13 provided in the end part 12 is an inlet for the vitreous bodies etc. 50 and is therefore preferably provided as close to the vitreous bodies etc. 50 to be collected. Therefore, while the end part 12 is made to have a smaller diameter than the probe main body 11, the vitreous body surgical probe 10 has the end part 12 with a small diameter connected to the probe main body 11, which is why the probe main body 11 and the end part 12 typically have differing inner diameters. Accordingly, in order to make it possible for the cutter 20 to slide along the respective inner surfaces of the probe main body 11 and the end part 12, the cutter 20 is squeezed and reduced in diameter, so as for the diameter of the cutter 20 to match to the respective inner diameters of the probe main body 11 and the end part 12. Note that there are times when the cutter 20 rotates within the probe. Therefore, the central axes of the probe main body 11 and the end part 12 should be matched to each other.

The vitreous bodies etc. 50 that are cut into small pieces are sucked in at the back side (left side of the drawing) of the vitreous body surgical probe 10 and collected. At this time, since the probe main body 11 may be a pipe having the same thickness as conventional products, it has the same suction force as in the past, and the same collecting ability as in the past can be assured while the vitreous bodies etc. 50 do not clog up the probe much at the time of suctioning. Note that the bending rigidity of the probe main body 11 is also the same as the conventional products.

FIG. 4 shows cross-sections illustrating an embodiment of another vitreous body surgical probe, wherein FIG. 4(a) illustrates a state where a cutter is pulled back, and FIG. 4(b) illustrates a state where a vitreous body or the like is cut. Note that the end part 12 of a vitreous body surgical probe 10a illustrated in the drawing here has a smaller diameter than the probe main body 11 but the same inner diameter as the probe main body 11.

The structure of the vitreous body surgical probe 10a as illustrated in FIG. 4 cannot be manufactured by joining pipes made of different materials to the probe main body 11 and the end part 12, respectively. However, it can be manufactured if the end part 12 is formed by cutting a material with a thick wall.

Use of the vitreous body surgical probe 10a has an advantage that it is easier to manufacture the cutter 20 than with the vitreous body surgical probe illustrated in FIG. 3 since the cutter 20 can have a fixed diameter.

### Explanation of References

10, 10a: Vitreous body surgical probe
11: Probe main body
12: End part
13: Opening
14: Tip surface
20: Cutter
21: Blade part
50: Vitreous body etc.

## Claims

1. A vitreous body surgical probe (10), comprising:
a probe main body (11) having a pipe shape; and
an end part (12) having an approximately tube shape, which is connected to the very end part of the probe main body (11), has a smaller diameter than the probe main body (11), is shorter than the probe main body (11), and includes a sealed tip surface and an opening in the side;
wherein the vitreous body surgical probe (10) further comprises a cutter (20), which has two outer diameters that match the respective inner diameters of the probe main body (11) and the end part (12), has a blade part (21) on a tip, and slides while touching inner surfaces of both the probe main body (11) and the end part (12).

## Patentansprüche

1. Chirurgische Glaskörpersonde (10), umfassend:
einen Sondenhauptkörper (11), der eine Rohrform aufweist; und
einen Endteil (12), der annähernd eine Rohrform aufweist und mit dem äußersten Endteil des Sondenhauptkörpers (11) verbunden ist, einen kleineren Durchmesser als der Sondenhauptkörper (11) aufweist, kürzer als der Sondenhauptkörper (11) ist und eine versiegelte Spitzenfläche und eine Öffnung an der Seite aufweist;
wobei die chirurgische Glaskörpersonde (10) des Weiteren eine Schneidvorrichtung (20) umfasst, die zwei Außendurchmesser aufweist, die mit den jeweiligen Innendurchmessern des Sondenhauptkörpers (11) und des Endteils (12) übereinstimmen, einen Klingenteil (21) an einer Spitze aufweist und gleitet, während sie Innenflächen sowohl des Sondenhauptkörpers (11) als auch des Endteils (12) berührt.

## Revendications

1. Sonde chirurgicale pour corps vitré (10), comprenant :
un corps principal de sonde (11) ayant la forme d'un tuyau ; et
une partie terminale (12) de forme approximativement tubulaire, qui est reliée à la partie située tout au bout du corps principal de sonde (11), a un diamètre inférieur à celui du corps principal de sonde (11), est plus courte que le corps principal de sonde (11), et comporte une surface de pointe scellée et une ouverture sur le côté ;
dans laquelle la sonde chirurgicale pour corps vitré (10) comprend en outre un dispositif de coupe (20), qui possède deux diamètres extérieurs qui correspondent aux diamètres intérieurs respectifs du corps principal de sonde (11) et de la partie terminale (12), qui possède une partie lame (21) sur une pointe, et qui coulisse tout en touchant des surfaces intérieures du corps principal de sonde (11) ainsi que de la partie terminale (12).
